# EUROPEAN PATENT APPLICATION

(11) **EP 3 804 763 A1**
(43) Date of publication of application: **14.04.2021**
(21) Application number: 19810117.2
(22) Date of filing: 30.05.2019
(51) Int. Cl.: A61K 47/61, A61K 48/00, A61P 37/06

(54) **PHARMACEUTICAL COMPOSITION CONTAINING COMPLEX OF CHEMICALLY-MODIFIED SIRNA AND SCHIZOPHYLLAN**

(30) Priority: 30.05.2018 JP 2018103933
(71) Applicant: NapaJen Pharma, Inc., Burlingame, California 94010 (US)
(72) Inventor: HIGUCHI, Sadaharu, Koganei-shi, Tokyo 184-0012 (JP); UNO, Atsushi, Koganei-shi, Tokyo 184-0012 (JP); ANDO, Hironori, Koganei-shi, Tokyo 184-0012 (JP)
(74) Representative: Müller-Boré & Partner Patentanwälte PartG mbB
(86) International application number: PCT/JP2019/021563
(87) International publication number: WO 2019/230898

(57) **Abstract**

The purpose of the present invention is to provide chemically-modified siRNA having polydeoxyadenylic acid added to the 5' end of the sense strand thereof, wherein when complexed with schizophyllan, the chemically-modified siRNA is highly resistant to RNase and effectively exhibits RNAi activity. The problem is solved by modified siRNA having polydeoxyadenylic acid at the 5' end of the sense strand thereof, wherein a particular chemical modification is applied to CA, UA, and UG dinucleotide sequences in the base sequence of the sense strand thereof, and CA, UA, and UG dinucleotide sequences in the base sequence beyond the eighth base from the 5' end of the antisense strand thereof.

## Description

### TECHNICAL FIELD

The present invention relates to a pharmaceutical composition containing a complex of a chemically modified siRNA and schizophyllan. More specifically, the present invention relates to a pharmaceutical composition containing a complex of a chemically modified siRNA having polydeoxyadenylic acid added to the 5' end of the sense strand thereof and schizophyllan, the composition being highly resistant to RNase and effectively exhibiting RNAi activity.

### BACKGROUND ART

RNA interference (RNAi) discovered in 1998, with magnitude and persistence of its effect being remarkably superior to the conventional antisense method, is so epoch-making gene expression-inhibiting method that its medicinal application has been expected. However, because a double-stranded RNA exhibiting RNAi activity (that is, siRNA) is often degraded in the course from administration to uptake by a target cell or in the cell, it has been difficult to form an RISC complex as its active body in the cell.

Unmodified siRNAs designed according to a suitable algorithm are so extremely easily degraded with RNase present in blood, for example, due to their in vivo and intracellular fragility to achieve the expected RNAi activity, that only a few RNAi effect can be exerted in a target cell. Accordingly, it has been conventionally proposed to apply various chemical modifications to siRNAs so as to impart RNase resistance (Non-Patent Documents 1 to 3). However, most siRNAs developed as nucleic acid drugs have chemical modifications applied to all the bases, leading to the failure to achieve the RNAi activity expected for unmodified siRNAs at the initial sequence design. That is, at present, a chemically modified siRNA having excellent RNase resistance with a small amount of chemical modification and inducing RNAi activity as originally expected has not been found (Non-Patent Document 4).

On the other hand, as an siRNA delivery technology, an siRNA/SPG complex of an siRNA having polydeoxyadenylic acid added thereto and schizophyllan (SPG) has been proposed (see Patent Document 1). The siRNA/SPG complex can selectively deliver the siRNA to Dectin-1-expressing cells such as dendritic cells, and once delivered into the cells, the RNAi effect can be effectively exerted. Therefore, the complex is expected for practical use as a nucleic acid drug. Because the siRNA/SPG complex is in a state where the siRNA is embedded in SPG, some degree of RNase resistance can be expected, but degradation with RNase is unavoidable in blood where a large amount of RNase is present. In order to develop the siRNA/SPG complex as a nucleic acid drug, it is desired to develop a technique for improving the resistance to RNase while maintaining the RNAi activity.

### PRIOR ART DOCUMENTS

### NON-PATENT DOCUMENTS

Non-Patent Document 1: Development of Therapeutic-Grade Small Interfering RNAs by Chemical Engineering, Jesper B. Bramsen and Jorgen Kjems, Front Genet. 2012; 3: 154.
Non-Patent Document 2: Oligonucleotide Therapies: The Past and the Present, Karin E. Lundin, Olof Gissberg, and C.I. Edvard Smith, Hum Gene Ther. 2015 Aug 1; 26(8): 475-485.
Non-Patent Document 3: siRNAmod: A database of experimentally validated chemically modified siRNAs, Showkat Ahmad Dar, Anamika Thakur, Abid Qureshi & Manoj Kumar, Sci Rep.2016; 6: 20031.
Non-Patent Document 4: Preclinical and clinical development of siRNA-based therapeutics, Gulnihal Ozcan, Bulent Ozpolat, Robert L. Coleman, Anil K. Sood, and Gabriel Lopez-Berestein, Adv Drug Deliv Rev. 2015 June 29; 87: 108-119.

### PATENT DOCUMENT

Patent Document 1: WO 2009/078470 A

### SUMMARY OF THE INVENTION

### PROBLEMS TO BE SOLVED BY THE INVENTION

An object of the present invention is to provide a pharmaceutical composition containing a complex of a chemically modified siRNA having polydeoxyadenylic acid (unless otherwise specified, hereinafter used to mean to also include phosphorothioated polydeoxyadenylic acid) added to the 5' end of the sense strand thereof and schizophyllan, the pharmaceutical composition being highly resistant to RNase and effectively exhibiting RNAi activity.

### MEANS FOR SOLVING THE PROBLEM

When chemically modifying an siRNA having polydeoxyadenylic acid added to the 5' end of the sense strand thereof to improve the resistance to RNase, a person skilled in the art would usually consider it essential to enhance the resistance to RNase by chemical modification at the following two locations: (1) the side of the 5' end of the antisense strand, which is said to have a particularly weak bond with the sense strand, and (2) the neighborhood of the connection point between the siRNA and polydeoxyadenylic acid. The present inventors have confirmed that even when these two locations are chemically modified, the resistance to RNase is not sufficiently improved. The inventors have further examined chemical modification methods for siRNA reported in publicly known documents.

Under such circumstances, the present inventors, through intensive studies, have found that even with relatively few chemical modifications, applying a specific chemical modification to a specific base in an siRNA having polydeoxyadenylic acid added to the 5' end of the sense strand thereof can markedly improve the resistance to RNase for the siRNA having polydeoxyadenylic acid added to the 5' end of the sense strand thereof when complexed with schizophyllan, compared to a normal siRNA having no polydeoxyadenylic acid added, leading to effective exhibition of RNAi activity. The present invention has been completed by further conducting studies based on the above findings.

In other words, the present invention provides an invention of the aspects described below. The modification method of the present invention represented by conditions (i) to (ix) in item 1 below is also referred to as A-3 modification in the present specification.
Item 1. A pharmaceutical composition containing a schizophyllan/chemically modified siRNA complex in which a chemically modified siRNA is complexed with schizophyllan:
   wherein in the chemically modified siRNA, polydeoxyadenylic acid is added to the 5' end of the sense strand,
   the base sequence of the sense strand of the chemically modified siRNA contains at least one dinucleotide sequence selected from the group consisting of CA, UA and UG,
   the base sequence of the 8th base and the subsequent bases from the 5' end of the antisense strand of the chemically modified siRNA contains at least one dinucleotide sequence selected from the group consisting of CA, UA and UG, and
   the chemically modified siRNA satisfies the following conditions (i) to (ix):
      (i) in the base sequence of the sense strand, when a dinucleotide sequence composed of CA is contained, the hydroxy group at the 2' position of the cytidylic acid residue in the dinucleotide sequence is replaced with a fluoro group, and the hydroxy group at the 2' position of the adenylic acid residue in the dinucleotide sequence is replaced with a methoxy group,
      (ii) in the base sequence of the sense strand, when a dinucleotide sequence composed of UA is contained, the hydroxy group at the 2' position of the uridylic acid residue in the dinucleotide sequence is replaced with a fluoro group, and the hydroxy group at the 2' position of the adenylic acid residue in the dinucleotide sequence is replaced with a methoxy group,
      (iii) in the base sequence of the sense strand, when a dinucleotide sequence composed of UG is contained, the hydroxy group at the 2' position of the uridylic acid residue in the dinucleotide sequence is replaced with a fluoro group, and the hydroxy group at the 2' position of the guanylic acid residue in the dinucleotide sequence is replaced with a methoxy group,
      (iv) in the base sequence of the 8th base and the subsequent bases from the 5' end of the antisense strand, when a dinucleotide sequence composed of CA is contained, the hydroxy group at the 2' position of the cytidylic acid residue in the dinucleotide sequence is replaced with a fluoro group, and the hydroxy group at the 2' position of the adenylic acid residue in the dinucleotide sequence is replaced with a methoxy group,
      (v) in the base sequence of the 8th base and the subsequent bases from the 5' end of the antisense strand, when a dinucleotide sequence composed of UA is contained, the hydroxy group at the 2' position of the uridylic acid residue in the dinucleotide sequence is replaced with a fluoro group, and the hydroxy group at the 2' position of the adenylic acid residue in the dinucleotide sequence is replaced with a methoxy group,
      (vi) in the base sequence of the 8th base and the subsequent bases from the 5' end of the antisense strand, when a dinucleotide sequence composed of UG is contained, the hydroxy group at the 2' position of the uridylic acid residue in the dinucleotide sequence is replaced with a fluoro group, and the hydroxy group at the 2' position of the guanylic acid residue in the dinucleotide sequence is replaced with a methoxy group,
      (vii) the 1st to 7th ribonucleotide residues from the 5' end of the antisense strand are not chemically modified,
      (viii) when chemical modification is performed according to the above (i) to (vi), in a case where neither the 1st ribonucleotide residue from the 5' end of the sense strand nor the 19th ribonucleotide residue from the 5' end of the antisense strand is chemically modified, if the 1st ribonucleotide residue from the 5' end of the sense strand is a cytidylic acid residue or a uridylic acid residue, the hydroxy group at the 2' position is replaced with a fluoro group, and if the ribonucleotide residue is an adenylic acid residue or a guanylic acid residue, the hydroxy group at the 2' position is replaced with a methoxy group,
      (ix) when the chemical modification according to the above (i) to (vi) is performed, in a case where both of the 1st ribonucleotide residue from the 5' end of the sense strand and the 19th ribonucleotide residue from the 5' end of the antisense strand are chemically modified, the 19th ribonucleotide residue from the 5' end of the antisense strand is not chemically modified.
Item 2. The pharmaceutical composition according to Item 1, wherein the polydeoxyadenylic acid added to the 5' end of the sense strand of the chemically modified siRNA has a length of 10 to 100 bases.
Item 3. The pharmaceutical composition according to Item 1 or 2, wherein the siRNA is an siRNA for CD40.
Item 4. The pharmaceutical composition according to any one of Items 1 to 3, wherein the chemically modified siRNA contains the following sense strand and antisense strand.
   Sense strand: 5'-C(F)A(M)GGAGACCU(F)G(M)GC(F)A(M)CU(F)G(M)GAdtdt-3'
   Antisense strand: 5'-UCCAGUGCC(F)A(M)GGUCUCCU(F)Gdtdt-3'
   [In the sense strand and the antisense strand, "dt" refers to a deoxythymidylic acid residue, "U(F)" refers to a uridylic acid residue in which the hydroxy group at the 2' position is modified with a fluoro group, "G(M)" refers to a guanylic acid residue in which the hydroxy group at the 2' position is replaced with a methoxy group, "C(F)" refers to a cytidylic acid residue in which the hydroxy group at the 2' position is modified with a fluoro group, and "A(M)" refers to an adenylic acid residue in which the hydroxy group at the 2' position is replaced with a methoxy group.]
Item 5. The pharmaceutical composition according to Item 4, wherein the polydeoxyadenylic acid added to the 5' end of the sense strand has a length of 40 bases.
Item 6. The pharmaceutical composition according to any one of Items 1 to 3, wherein the chemically modified siRNA contains the following sense strand and antisense strand.
   Sense strand: 5'-G(M)A(M)C(F)A(M)GAAACU(F)G(M)GU(F)G(M)AGU(F)G(M)Adtdt-3 '
   Antisense strand: 5'-UCACUCACC(F)A(M)GUUUCU(F)G(M)UCdtdt-3'
   [In the sense strand and the antisense strand, "dt" refers to a deoxythymidylic acid residue, "U(F)" refers to a uridylic acid residue in which the hydroxy group at the 2' position is modified with a fluoro group, "G(M)" refers to a guanylic acid residue in which the hydroxy group at the 2' position is replaced with a methoxy group, "C(F)" refers to a cytidylic acid residue in which the hydroxy group at the 2' position is modified with a fluoro group, and "A(M)" refers to an adenylic acid residue in which the hydroxy group at the 2' position is replaced with a methoxy group.]
Item 7. The pharmaceutical composition according to Item 6, wherein the polydeoxyadenylic acid added to the 5' end of the sense strand has a length of 40 bases.
Item 8. The pharmaceutical composition according to any one of Items 1 to 3, wherein the chemically modified siRNA contains the following sense strand and antisense strand.
   Sense strand: 5'-A(M)GU(F)G(M)U(F)G(M)GCC(F)A(M)CGU(F)G(M)GGC(F)A(M)Adtdt-3'
   Antisense strand: 5'-UUGCCCACGU(F)G(M)GCC(F)A(M)C(F)A(M)CUdtdt-3'
   [In the sense strand and the antisense strand, "dt" refers to a deoxythymidylic acid residue, "U(F)" refers to a uridylic acid residue in which the hydroxy group at the 2' position is modified with a fluoro group, "G(M)" refers to a guanylic acid residue in which the hydroxy group at the 2' position is replaced with a methoxy group, "C(F)" refers to a cytidylic acid residue in which the hydroxy group at the 2' position is modified with a fluoro group, and "A(M)" refers to an adenylic acid residue in which the hydroxy group at the 2' position is replaced with a methoxy group.]
Item 9. The pharmaceutical composition according to Item 8, wherein the polydeoxyadenylic acid added to the 5' end of the sense strand has a length of 40 bases.
Item 10. The pharmaceutical composition according to any one of Items 1 to 3, wherein the chemically modified siRNA contains the following sense strand and antisense strand.
   Sense strand: 5'-C(F)AGA(M)AAC(F)A(M)GUUC(F)A(M)CCUU(F)G(M)Adtdt-3 '
   Antisense strand: 5'-UCAAGGU(F)G(M)AACU(F)G(M)UUUCU(F)Gdtdt-3'
   [In the sense strand and the antisense strand, "dt" refers to a deoxythymidylic acid residue, "U(F)" refers to a uridylic acid residue in which the hydroxy group at the 2' position is modified with a fluoro group, "G(M)" refers to a guanylic acid residue in which the hydroxy group at the 2' position is replaced with a methoxy group, "C(F)" refers to a cytidylic acid residue in which the hydroxy group at the 2' position is modified with a fluoro group, and "A(M)" refers to an adenylic acid residue in which the hydroxy group at the 2' position is replaced with a methoxy group.]
Item 11. The pharmaceutical composition according to Item 10, wherein the polydeoxyadenylic acid added to the 5' end of the sense strand has a length of 40 bases.
Item 12. The pharmaceutical composition according to any one of Items 1 to 11, being a liquid preparation further containing sodium chloride, potassium dihydrogen phosphate and disodium hydrogen phosphate.
Item 13. The pharmaceutical composition according to any one of Items 3 to 12, being used for treatment or prevention of resistance or rejection to a transplanted organ, transplanted tissue or transplanted cell.
Item 14. The pharmaceutical composition according to any one of Items 3 to 12, being used for treatment or prevention of graft-versus-host disease.
Item 15. A method for treating or preventing resistance or rejection to a transplanted organ, transplanted tissue or transplanted cell, including administering to a patient undergoing an organ, tissue or cell transplantation treatment the pharmaceutical composition according to any one of Items 3 to 12 before and/or after the transplantation treatment.
Item 16. A method for treating or preventing graft-versus-host disease, including administering to a patient undergoing an allogeneic hematopoietic stem cell transplantation treatment the pharmaceutical composition according to any one of Items 3 to 12 before and/or after the transplantation treatment.
Item 17. A use of the pharmaceutical composition according to any one of Items 3 to 12 for production of an agent for treating or preventing resistance or rejection to a transplanted organ, transplanted tissue or transplanted cell.
Item 18. A use of the pharmaceutical composition according to any one of Items 3 to 12 for production of an agent for treating or preventing graft-versus-host disease.

### ADVANTAGES OF THE INVENTION

The pharmaceutical composition of the present invention contains a complex of a specific chemically modified siRNA having polydeoxyadenylic acid added to the 5' end of the sense strand thereof and schizophyllan, so that the composition can be highly resistant to RNase and effectively exhibit RNAi activity.

In particular, in a pharmaceutical composition containing a complex of an siRNA having a predetermined chemical modification for CD40 and schizophyllan, the resistance to RNase and formulation stability are so high that the composition is useful for treatment or prevention of the resistance or rejection to a transplanted organ, transplanted tissue or transplanted cell, graft-versus-host disease, and the like.

### BRIEF DESCRIPTION OF THE DRAWINGS

Fig. 1 shows the results of evaluation of the gene expression inhibitory effect for various chemically modified siRNAs and SPG/chemically modified siRNA complexes obtained by using chemically modified siRNAs in Reference Test Example 1.
Fig. 2 shows the results of evaluation of stability in serum for various chemically modified siRNAs and SPG/chemically modified siRNA complexes obtained by using chemically modified siRNAs in Reference Test Example 2.
Fig. 3 shows the results of evaluation of the gene expression inhibitory effect for SPG/chemically modified siRNA complexes obtained by using various chemically modified siRNAs in Reference Test Example 3.
Fig. 4 shows the results of evaluation of the stability in serum for SPG/chemically modified siRNA complexes obtained by using various chemically modified siRNAs in Reference Test Example 4.
Fig. 5 shows the results of evaluation of the gene expression inhibitory effect for various chemically modified siRNAs and SPG/chemically modified siRNA complexes obtained by using chemically modified siRNAs in Test Example 1.
Fig. 6 shows the results of evaluation of the stability in serum for SPG/chemically modified siRNA complexes obtained by using various modified siRNAs in Test Example 2.
Fig. 7 shows the results of evaluation of the cell growth suppressing effect by an ex vivo MLR (mixed lymphocyte culture) assay for SPG/chemically modified siRNA complexes obtained by using various modified siRNAs in Test Example 3.
Fig. 8 shows the results of evaluation of the gene expression inhibitory effect for various chemically modified siRNAs and SPG/chemically modified siRNA complexes obtained by using chemically modified siRNAs in Test Example 4.
Fig. 9 shows the results of evaluation of the life-prolonging effect by administration of SPG/chemically modified siRNA complexes prepared using chemically modified siRNAs for CD40 in bone marrow cell transplantation using aGVHD model mice in Test Example 7.

### EMBODIMENTS OF THE INVENTION

In the present invention, when a base sequence composed of two or more bases is shown, the left end of the base sequence is the 5' end and the right end is the 3' end.

The pharmaceutical composition of the present invention is characterized by containing a schizophyllan/chemically modified siRNA complex in which a specific chemically modified siRNA is complexed with schizophyllan. Hereinafter, a description is made of the pharmaceutical composition of the present invention.

### 1. Chemically modified siRNA

### Base sequence and structure of siRNA having polydeoxyadenylic acid added

In the present invention, a chemically modified siRNA is used, in which polydeoxyadenylic acid is added to the 5' end of the sense strand, the sense strand and the antisense strand contain a specific dinucleotide sequence, and a specific chemical modification is applied to the dinucleotide sequence.

In the chemically modified siRNA, the base sequences of the sense strand and the antisense strand are set depending on the target sequence in a target gene. The target sequence in the target gene can be set by a publicly known technique according to the manual (Dicer Substrate RNAi Design) of IDT (Integrated DNA Technologies, INC) or the like. Usually, an siRNA is designed to contain a sequence that is 100% identical to the target sequence or a sequence in which one or several bases have been substituted/added from the target sequence as long as a desired RNA interference effect can be obtained. It has also been reported that an siRNA having an excellent RNA interference effect can be designed by designing a double-stranded RNA in which the 5' end of the antisense strand is an A/U pair, the 5' end of the sense strand is a G/C pair, about 5 A/U pairs are present on the side of the 5' end of the antisense strand, and 9 or more G/C pairs are absent in the double strand (Ui-Tei et al, Nucleic Acids Res., 32, 936-948 (2004)). Examples of an siRNA for CD40 include an siRNA having the sense strand composed of the base sequence represented by SEQ ID NO: 1 and the antisense strand composed of the base sequence represented by SEQ ID NO: 2, an siRNA having the sense strand composed of the base sequence represented by SEQ ID NO: 3 and the antisense strand composed of the base sequence represented by SEQ ID NO: 4, an siRNA having the sense strand composed of the base sequence represented by SEQ ID NO: 5 and the antisense strand composed of the base sequence represented by SEQ ID NO: 6, an siRNA having the sense strand composed of the base sequence represented by SEQ ID NO: 7 and the antisense strand composed of the base sequence represented by SEQ ID NO: 8, and an siRNA having the sense strand composed of the base sequence represented by SEQ ID NO: 9 and the antisense strand composed of the base sequence represented by SEQ ID NO: 10.

In the chemically modified siRNA, the base sequence of the sense strand contains at least one dinucleotide sequence selected from the group consisting of CA, UA and UG, and the base sequence of the 8th base and the subsequent bases from the 5' end of the antisense strand contains at least one dinucleotide sequence selected from the group consisting of CA, UA and UG. By subjecting such dinucleotide sequences to a specific chemical modification as described later, it becomes possible to improve the resistance to RNase while maintaining the RNAi activity when complexed with SPG.

The target gene for the chemically modified siRNA is not particularly limited, but may be appropriately selected based on the use of the chemically modified siRNA. From the viewpoint of use in medicinal applications, a gene which is involved in the pathological condition and the expression of which is desired to be inhibited is suitable.

A preferred aspect of the target gene for the chemically modified siRNA includes a gene that is expressed in a Dectin-1 expressing cell and affects the in vivo function of the cell. Dectin-1 is a receptor having a C-type lectin-type sugar chain recognition domain (pattern recognition receptor) present on the cell membrane. Dectin-1 has a region that specifically recognizes SPG outside the cell, and has a motif that transmits an activation signal called ITAM (immunoreceptor tyrosinase-based activation motif-1) inside the cell. When Dectin-1 recognizes SPG, it promotes production of NF-κB and inflammatory cytokine, inducing a biological defense response. Specific examples of the Dectin-1 expressing cell include macrophages, dendritic cells and neutrophils. SPG has a β-1,3-glucan structure and is known to be delivered by endocytosis into the Dectin-1 expressing cell by binding to Dectin-1 present on the cell membrane of the Dectin-1 expressing cell. When the chemically modified siRNA is complexed with SPG, recognition of SPG by Dectin-1 enables the chemically modified siRNA to be selectively delivered into the Dectin-1 expressing cell. In addition, by selecting a gene that affects the in vivo function of the Dectin-1-expressing cell as the target gene for the chemically modified siRNA, it becomes possible to induce in vivo immunosuppression, regulating the immunity.

When the chemically modified siRNA is expressed in the Dectin-1 expressing cell and its target gene is a gene that affects the in vivo function of the cell, the type of the gene is not particularly limited, but from the viewpoint of more effectively inducing in vivo immunomodulation, especially immunosuppression, suitable target genes include a gene related to antigen presentation, such as a gene coding a co-stimulatory factor (also referred to as a co-stimulatory molecule) such as CD40.

In the chemically modified siRNA, the base lengths of the sense strand and the antisense strand are not particularly limited, but preferably 21.

In addition, in the chemically modified siRNA, the sense strand and the antisense strand may hybridize to each other to form a double strand so as to have a dangling end composed of about 2 to 5 ribonucleotides or deoxyribonucleotides at each 3' end.

An example of the preferred aspect of the chemically modified siRNA includes one in which both the sense and antisense strands have a base length of 21, and a dangling end is formed composed of two ribonucleotides or deoxyribonucleotides at the 5' end of the sense strand and the 3' end of the antisense strand. That is, in the case of such an siRNA, the sequence of the 3rd to 21st ribonucleotides from the 3' end of the antisense strand is complementary to the sequence of the 1st to 19th ribonucleotides from the 5' end of the sense strand.

In the chemically modified siRNA, polydeoxyadenylic acid is added to the 5' end of the sense strand.

The polydeoxyadenylic acid forms a triple helical structure with two units of SPG, playing a role of complexing the chemically modified siRNA with SPG.

The number of deoxyadenylic acid residues composing polydeoxyadenylic acid is not particularly limited, as long as it is possible to form a complex with SPG, but may be, for example, 10 to 100, preferably 20 to 100, more preferably 20 to 80, still more preferably 30 to 50, particularly preferably 40.

In the chemically modified siRNA, polydeoxyadenylic acid is preferably directly bonded to the 5' end of the sense strand by phosphodiester bond, but may be bonded to the 5' end of the sense strand via a linker (spacer).

In addition, in the chemically modified siRNA, the phosphodiester bond of the sense strand, the antisense strand and polydeoxyadenylic acid may be partially or entirely phosphorothioated (S-converted). The polydeoxyadenylic acid moiety of the chemically modified siRNA is preferably S-converted. The S-converted phosphodiester bond is a bond structure in which one of the oxygen atoms in the phosphate residue of the phosphodiester bond portion is replaced with a sulfur atom.

### Chemical modification of siRNA

In the chemically modified siRNA used in the present invention, the sense strand and the antisense strand have undergone chemical modification (A-3 modification) according to the conditions (i) to (ix) described below. In the chemically modified siRNA, it is necessary that the sense strand and the antisense strand be not chemically modified except for the conditions (i) to (ix) described below.

### <Chemical modification of sense strand>

The sense strand of the chemically modified siRNA is chemically modified so as to satisfy the following conditions (i) to (iii).
(i) In the base sequence of the sense strand, when a dinucleotide sequence composed of CA is contained, the hydroxy group at the 2' position of the cytidylic acid residue in the dinucleotide sequence is replaced with a fluoro group, and the hydroxy group at the 2' position of the adenylic acid residue in the dinucleotide sequence is replaced with a methoxy group.
(ii) in the base sequence of the sense strand, when a dinucleotide sequence composed of UA is contained, the hydroxy group at the 2' position of the uridylic acid residue in the dinucleotide sequence is replaced with a fluoro group, and the hydroxy group at the 2' position of the adenylic acid residue in the dinucleotide sequence is replaced with a methoxy group,
(iii) in the base sequence of the sense strand, when a dinucleotide sequence composed of UG is contained, the hydroxy group at the 2' position of the uridylic acid residue in the dinucleotide sequence is replaced with a fluoro group, and the hydroxy group at the 2' position of the guanylic acid residue in the dinucleotide sequence is replaced with a methoxy group,

That is, for example, when the sense strand of the siRNA is a base sequence with 21 base length as shown in (A) of Table 1, the one that has undergone the chemical modification according to the conditions (i) to (iii) includes the aspect as shown in (B) of Table 1.

**[Table 1]**

| | |
|---|---|
| (A) Sense strand before chemical modification | 5'-CGUGCAGUGACAAACAGUAdtdt-3' |
| ↓ | |
| (B) Sense strand after chemical modification according to conditions (i) to (iii) | 5'-CGU(F)G(M)C(F)A(M)GU(F)G(M)AC(F)A(M)AAC(F)A(M)GU(F)A(M)dtdt-3' |

In the Table, "dt" refers to a deoxythymidylic acid residue, "U(F)" refers to a uridylic acid residue in which the hydroxy group at the 2' position is modified with a fluoro group, "G(M)" refers to a guanylic acid residue in which the hydroxy group at the 2' position is replaced with a methoxy group, "C(F)" refers to a cytidylic acid residue in which the hydroxy group at the 2' position is modified with a fluoro group, and "A(M)" refers to an adenylic acid residue in which the hydroxy group at the 2' position is replaced with a methoxy group.

### <Chemical modification of antisense strand>

The antisense strand of the chemically modified siRNA is chemically modified so as to satisfy the following conditions (iv) to (vii).
(iv) In the base sequence of the 8th base and the subsequent bases from the 5' end of the antisense strand, when a dinucleotide sequence composed of CA is contained, the hydroxy group at the 2' position of the cytidylic acid residue in the dinucleotide sequence is replaced with a fluoro group, and the hydroxy group at the 2' position of the adenylic acid residue in the dinucleotide sequence is replaced with a methoxy group,
(v) in the base sequence of the 8th base and the subsequent bases from the 5' end of the antisense strand, when a dinucleotide sequence composed of UA is contained, the hydroxy group at the 2' position of the uridylic acid residue in the dinucleotide sequence is replaced with a fluoro group, and the hydroxy group at the 2' position of the adenylic acid residue in the dinucleotide sequence is replaced with a methoxy group,
(vi) in the base sequence of the 8th base and the subsequent bases from the 5' end of the antisense strand, when a dinucleotide sequence composed of UG is contained, the hydroxy group at the 2' position of the uridylic acid residue in the dinucleotide sequence is replaced with a fluoro group, and the hydroxy group at the 2' position of the guanylic acid residue in the dinucleotide sequence is replaced with a methoxy group,
(vii) the 1st to 7th ribonucleotide residues from the 5' end of the antisense strand are not chemically modified.

That is, for example, when the antisense strand of the siRNA is a base sequence with 21 base length as shown in (A) of Table 2, the one that has undergone the chemical modification according to the conditions (iv) to (vii) includes the aspect as shown in (B) of Table 2.

**[Table 2]**

| | |
|---|---|
| (A) Antisense strand before chemical modification | 5' -UACUGUUUGUCACUGCACGdtdt-3' |
| ↓ | |
| (B) Antisense strand after chemical modification according to conditions (iv) to (vii) | 5' -UACUGUUU(F)G(M)UC(F)A(M)CU(F)G(M)C(F)A(M)CGdtdt-3' |

In the Table, "dt", "U(F)", "G(M)" and "A(M)" are as defined in Table 1

### <Chemical modification in the vicinity of polydeoxyadenylic acid>

In the chemically modified siRNA, the 1st ribonucleotide residue from the 5' end of the sense strand and the 19th ribonucleotide residue from the 5' end of the antisense strand are located closest to polydeoxyadenylic acid. In the chemically modified siRNA, the above conditions (i) to (vii) should not be applied to these ribonucleotide residues as they are in some cases.

Specifically, in the chemically modified siRNA, the sense strand and the antisense strand are chemically modified according to the conditions (i) to (vii). However, when the chemical modification is performed according to these conditions, in a case where neither the 1st ribonucleotide residue from the 5' end of the sense strand nor the 19th ribonucleotide residue from the 5' end of the antisense strand is chemically modified, or where both of them are chemically modified, chemical modification according to the following conditions (viii) and (ix) is required.

(viii) When the chemical modification is performed according to the above (i) to (vi), in a case where neither the 1st ribonucleotide residue from the 5' end of the sense strand nor the 19th ribonucleotide residue from the 5' end of the antisense strand is chemically modified, if the 1st ribonucleotide residue from the 5' end of the sense strand is a cytidylic acid residue or a uridylic residue, the 2' position thereof is modified with a fluoro group, and if the ribonucleotide residue is an adenylic acid residue or a guanylic acid residue, the 2' position thereof is modified with a methoxy group. (ix) When the chemical modification according to the above (i) to (vi) is performed, in a case where both of the 1st ribonucleotide residue from the 5' end of the sense strand and the 19th ribonucleotide residue from the 5' end of the antisense strand are chemically modified, the 19th ribonucleotide residue from the 5' end of the antisense strand is not chemically modified.

### <Method for chemical modification>

A method for replacing (modifying) the hydroxy group at the 2' position of the ribose moiety of each of a cytidylic acid residue and a uridylic acid residue with a fluoro group, and a method for replacing (modifying) the hydroxy group at the 2' position of the ribose moiety of each of an adenylic acid residue and a guanylic acid residue with a methoxy group are publicly known. Accordingly, the chemically modified siRNA used in the present invention can be produced by a known technique.

### <Suitable example of chemically modified siRNA>

Among the chemically modified siRNAs used in the present invention, suitable specific examples of chemically modified siRNAs obtained by chemically modifying siRNAs for human CD40 include the followings (1) to (4). These chemically modified siRNAs can be particularly suitably used for treatment or prevention of resistance or rejection to a transplanted organ, transplanted tissue, or transplanted cell, graft-versus-host disease, and the like.
(1) Chemically modified siRNA having sense strand: 5'-C(F)A(M)GGAGACCU(F)G(M)GC(F)A(M)CU(F)G(M)GAdtdt-3' and antisense strand: 5'-UCCAGUGCC(F)A(M)GGUCUCCU(F)Gdtdt-3'
(2) Chemically modified siRNA having sense strand: 5'-G(M)A(M)C(F)A(M)GAAACU(F)G(M)GU(F)G(M)AGU(F)G(M)Adtdt-3' and antisense strand: 5'-UCACUCACC(F)A(M)GUUUCU(F)G(M)UCdtdt-3'
(3) Chemically modified siRNA having sense strand: 5'-A(M)GU(F)G(M)U(F)G(M)GCC(F)A(M)CGU(F)G(M)GGC(F)A(M)Adtd-3' and antisense strand: 5'-UUGCCCACGU(F)G(M)GCC(F)A(M)C(F)A(M)CUdtdt-3'
(4) Chemically modified siRNA having sense strand: 5'-C(F)AGA(M)AAC(F)A(M)GUUC(F)A(M)CCUU(F)G(M)Adtdt-3' and antisense strand: 5'-UCAAGGU(F)G(M)AACU(F)G(M)UUUCU(F)Gdtdt-3'

In the sense strands and the antisense strands of the (1) to (4), "dt" refers to a deoxythymidylic acid residue, "U(F)" refers to a uridylic acid residue in which the hydroxy group at the 2' position is modified with a fluoro group, "G(M)" refers to a guanylic acid residue in which the hydroxy group at the 2' position is replaced with a methoxy group, "C(F)" refers to a cytidylic acid residue in which the hydroxy group at the 2' position is modified with a fluoro group, and "A(M)" refers to an adenylic acid residue in which the hydroxy group at the 2' position is replaced with a methoxy group.

### 2. SPG/chemically modified siRNA complex

In the present invention, the chemically modified siRNA is complexed with SPG to be used in the state of a schizophyllan/chemically modified siRNA complex (SPG/chemically modified siRNA complex). SPG is a polysaccharide having a β-1,3-glucan structure, and the SPG/chemically modified siRNA complex undergoes endocytosis caused by binding of the SPG to the aforementioned cell surface receptor Dectin-1, allowing the complex to be delivered into the cell.

Schizophyllan (sometimes abbreviated as SPG), which is a component of the SPG/chemically modified siRNA complex, can be produced according to the standard method described in a document (A.C.S.38 (1), 253 (1997); Carbohydrate Research, 89, 121-135 (1981)). The schizophyllan obtained in this way can be subjected to ultrasonic treatment to yield a desired molecular weight of schizophyllan.

The molecular weight of schizophyllan used in the SPG/chemically modified siRNA complex is not particularly limited, but may be appropriately set depending on the chain length of polydeoxyadenylic acid added to the chemically modified siRNA, and the like. Specifically, the molecular weight of schizophyllan is usually 25,000 to 500,000, preferably 25,000 to 250,000.

The SPG/chemically modified siRNA complex can be prepared according to a publicly known method for complexing SPG with polydeoxyadenylic acid. Specifically, a method for production including the following steps (1) to (3) is exemplified: (1) preparing a chemically modified siRNA containing polydeoxyadenylic acid according to a publicly known method, (2) separately preparing SPG, and (3) then, using the polydeoxyadenylic acid bonded to the chemically modified siRNA and SPG to form a complex.

In the step (3) of the above method, the mixing ratio of the chemically modified siRNA and SPG can be appropriately selected depending on the chain length of polydeoxyadenylic acid and the molecular weight of SPG. In the SPG/chemically modified siRNA complex, one molecule of glucose in the main chain of SPG corresponds to one deoxyadenylic acid residue of polydeoxyadenylic acid, whereby one polydeoxyadenylic acid and two units of SPG take a triple helical structure. That is, in the SPG/chemically modified siRNA complex, polydeoxyadenylic acid is incorporated into a double helical structure formed of two units of SPG at one location or two or more locations to form a triple helical structure. For example, with a chemically modified siRNA containing polydeoxyadenylic acid having a base length of 40 and SPG having a molecular weight of 150,000, it is possible to take a triple helical structure including two molecules of SPG having a molecular weight of 150,000 and 17 molecules of chemically modified siRNA containing polydeoxyadenylic acid having the base length. It is preferable that a chemically modified siRNA containing polydeoxyadenylic acid be mixed with SPG at a molar ratio of 20 : 1 to 1 : 5, preferably 10 : 1 to 1 : 1, to complex a single strand polydeoxyadenylic acid region bonded to a chemically modified siRNA with SPG. By exposing a chemically modified siRNA containing polydeoxyadenylic acid and SPG at such a molar ratio under a complex formation condition, it is possible to make both of them efficiently interact with each other and thus improve the production efficiency of an SPG/chemically modified siRNA complex.

The triple helical structure of polydeoxyadenylic acid and SPG in an SPG/chemically modified siRNA complex can be specifically formed according to the following method. SPG takes a triple helical structure in nature or in water. This SPG is dissolved in a polar solvent such as DMSO (dimethylsulfoxide) or an alkaline aqueous solution such as a sodium hydroxide aqueous solution for denaturation to a single strand. Then, to the mixture, a chemically modified siRNA containing polydeoxyadenylic acid is added, and the solvent is replaced with water or the alkaline aqueous solution is neutralized (renewal process) to form a complexed triple helical structure (association structure) composed of a single strand moiety of polydeoxyadenylic acid linked to the chemically modified siRNA and two units of SPG. Such a complex of polydeoxyadenylic acid with a polysaccharide is considered to be formed mainly through hydrogen bonding and hydrophobic interaction.

### 3. Pharmaceutical composition

The pharmaceutical composition of the present invention includes the SPG/chemically modified siRNA complex. Because the SPG/chemically modified siRNA complex can be introduced into a cell, thereby inhibiting the expression of the target gene in the cell, the pharmaceutical composition of the present invention can be used for the purpose of inhibiting the expression of a target gene.

The pharmaceutical composition of the present invention can be prepared by containing a therapeutically effective amount of the SPG/chemically modified siRNA complex as an active component, and further combining a pharmaceutically acceptable carrier as appropriate. Examples of such a carrier include aqueous carriers such as purified water, sugar-containing aqueous solution, buffer solution, physiological saline and nuclease-free water; and excipients.

One aspect in a case where the pharmaceutical composition of the present invention is a liquid preparation used as an injection or the like includes a physiological saline containing a near-neutral phosphate buffer solution containing the SPG/chemically modified siRNA complex. In the aqueous solution, the concentration of each component is not particularly limited as long as it is substantially isotonic with the body fluid. For example, the solution includes the SPG/chemically modified siRNA complex in a concentration of 0.001 to 7 mg/mL, preferably 0.01 to 5 mg/mL of 2 to 10 mM phosphate buffer solution, preferably about 5 mM buffer solution in terms of the amount of chemically modified siRNA, has a near neutral pH, preferably a pH of 7.0 to 7.5, and is adjusted to be substantially isotonic with the body fluid with salt before use. This injection may be administered intravenously or the like as it is, or may be intravenously infused with Ringer's solution or the like.

The route of administration of the pharmaceutical composition of the present invention can be appropriately selected from conventionally used ways based on the symptom and condition of the patient, the type of the disease, and so on, including oral administration, parenteral administration (including intravenous, intraperitoneal, intramuscular, subcutaneous, intrarectal and intravaginal administrations), inhalation, systemic administration, topical administration (including external application to the skin or buccal cavity; and infusion into a site substantially free from invasion into a bloodstream, such as eyes, ears and nose).

When the target gene for the chemically modified siRNA contained in the pharmaceutical composition of the present invention is CD40, the pharmaceutical composition can be suitably used for treatment or prevention of resistance or rejection to a transplanted organ, transplanted tissue or transplanted cell (for example, kidney, heart, lung, bone marrow, skin, cornea, bone marrow cell) in organ, tissue or cell transplant therapy. When the pharmaceutical composition is used for treatment or prevention of resistance or rejection to a transplanted organ, transplanted tissue or transplanted cell, the pharmaceutical composition may be administered to a patient undergoing an organ, tissue or cell transplantation treatment before and/or after the transplantation treatment. Furthermore, when the target gene for the chemically modified siRNA contained in the pharmaceutical composition of the present invention is CD40, the pharmaceutical composition can be suitably used for treatment or prevention of graft-versus-host disease (GVHD). When the pharmaceutical composition is used for treatment or prevention of graft-versus-host disease (GVHD), the pharmaceutical composition may be administered to a patient undergoing an allogeneic hematopoietic stem cell transplantation treatment before and/or after the transplantation treatment.

When the target gene for the chemically modified siRNA contained in the pharmaceutical composition of the present invention is CD40, the dose of the pharmaceutical composition may be any amount as long as it is effective and non-toxic for the desired treatment or prevention. The dose can be determined by a person skilled in the art through routine experiments, and is not particularly limited, but for example, the dose of SPG/chemically modified siRNA complex per kg of body weight per day may be appropriately selected from the range of about 0.01 µg to 10 mg, preferably about 0.5 µg to 1 mg in terms of the amount of chemically modified siRNA.

### EXAMPLES

Hereinafter, a more detailed description is made of the present invention based on Examples, but the present invention is not limited thereto.

### Production Example: Production of SPG/chemically modified siRNA complex

In SPG/chemically modified siRNA complexes used in the following tests, all the component chemically modified siRNAs used were ones having S-converted polydeoxyadenylic acid (40 mer) added to the 5' end of the sense strand that had undergone the prescribed chemical modification.

The SPG/chemically modified siRNA complexes used in the following Test Examples were formed as follows. SPG having a molecular weight of about 150,000 was prepared in a 0.25 N sodium hydroxide aqueous solution at a final concentration of 15 mg/ml. Then, the solution was stirred by vibration for 1 hour and allowed to stand at 4°C for 1 day for denaturation. A solution of a chemically modified siRNA having a given sequence having S-converted polydeoxyadenylic acid added thereto dissolved in 330 mM of primary sodium phosphate was added to the denatured SPG solution for neutralization, and allowed to stand at 4°C for 24 hours or longer. At this time, adjustment was made to achieve 0.27 mol of SPG with respect to 1 mol of the chemically modified siRNA. The chemically modified siRNA having S-converted polydeoxyadenylic acid added thereto refers to one in which 40 deoxyadenylic acids are linked to the 5' end of the sense strand of the siRNA by phosphoester bonding.

### Reference Test Example 1

SPG/chemically modified siRNA complexes were prepared using chemically modified siRNAs (NJ050.11 and NJ050.13 shown in Table 3) for CD40. In addition, NJ050.12 and NJ050.13 shown in Table 3 were prepared as chemically modified siRNAs having no polydeoxyadenylic acid added thereto. The base sequences of the sense strand and antisense strand of the chemically modified siRNAs correspond to SEQ ID NOs: 1 and 2, respectively.

In NJ050.11, in the sense strand, the hydroxy group at the 2' position of each of all adenylic acid residues and guanylic acid residues is replaced with a methoxy group, and the hydroxy group at the 2' position of each of all cytidylic acid residues and uridylic acid residues is replaced with a fluoro group. In the antisense strand, the hydroxy group at the 2' position of each of adenylic acid residues and guanylic acid residues at the 8th base and the subsequent bases from the 5' end is replaced with a methoxy group, and the hydroxy group at the 2' position of each of cytidylic acid residues and uridylic acid residues at the 8th base and the subsequent bases from the 5' end is replaced with a fluoro group.

In NJ050.12, in both the sense strand and the antisense strand, the hydroxy group at the 2' position of each of cytidylic acid residues and uridylic acid residues in a dinucleotide sequence composed of CA, UA and UG is replaced with a fluoro group, and the hydroxy group at the 2' position of each of adenylic acid residues and guanylic acid residues in the dinucleotide sequence is replaced with a methoxy group.

In NJ050.13, in the sense strand, the hydroxy group at the 2' position of each of cytidylic acid residues and uridylic acid residues in a dinucleotide sequence composed of CA, UA and UG is replaced with a fluoro group, and the hydroxy group at the 2' position of each of adenylic acid residues and guanylic acid residues in the dinucleotide sequence is replaced with a methoxy group. In the antisense strand, the hydroxy group at the 2' position of each of adenylic acid residues and guanylic acid residues at the 8th base and the subsequent bases from the 5' end is replaced with a methoxy group, and the methoxy group at the 2' position of each of cytidylic acid residues and uridylic acid residues at the 8th base and the subsequent bases from the 5' end is replaced with a fluoro group.

**[Table 3]**

| NJ050.11 |
|---|
| Sense strand 5'→3':C(F)G(M)U(F)G(M)C(F)A(M)G(M)U(F)G(M)A(M)C(F)A(M)A(M)A(M)C(F)A(M)G(M)U(F)A(M)dtdt |
| Antisense strand 5'→3':UACUGU(F)U(F)U(F)G(M)U(F)C(F)A(M)C(F)U(F)G(M)C(F)A(M)C(F)G(M)dtdt |

| NJ050.12 |
|---|
| Sense strand 5'→3':CGU(F)G(M)C(F)A(M)GU(F)G(M)AC(F)A(M)AAC(F)A(M)GU(F)A(M)dtdt |
| Antisense strand 5'→3':U(F)A(M)CU(F)G(M)UUU(F)G(M)UC(F)A(M)CU(F)G(M)C(F)A(M)CGdtdt |

| NJ050.13 |
|---|
| Sense strand 5'→3':CGU(F)G(M)C(F)A(M)GU(F)G(M)AC(F)A(M)AAC(F)A(M)GU(F)A(M)dtdt |
| Antisense strand 5'→3':UACUGUUU(F)G(M)UC(F)A(M)CU(F)G(M)C(F)A(M)CGdtdt |

| |
|---|
| In the Table, "dt", "U(F)", "G(M)" and "A(M)" are as defined in Table 1. |

Each of the SPG/chemically modified siRNA complexes and the chemically modified siRNAs was introduced into 200,000 c-wrt-7LR cells (rat myelomonocytic leukemia-derived cells) by electroporation. After the introduction, the cells were seeded on a 48-well plate at 2 × 10⁵ cells/well, followed by incubation at 37°C under 5% CO2 for 2 hours. Then, lipopolysaccharide (LPS) was added to each well at 10 ng/mL, followed by incubation at 37°C under 5% CO2 for 22 hours. Then, the total RNA was extracted from the cells, and cDNA was synthesized from the total RNA. Using the synthesized cDNA as a template, real-time PCR was performed using a CD40 primer to measure the amount of CD40 mRNA. One subjected to the LPS treatment without insertion of siRNA was used as a positive control, and one subjected to neither the LPS treatment nor insertion of siRNA was used as a negative control. The amount of CD40 mRNA was corrected with the amount of 18S rRNA that is a housekeeping gene.

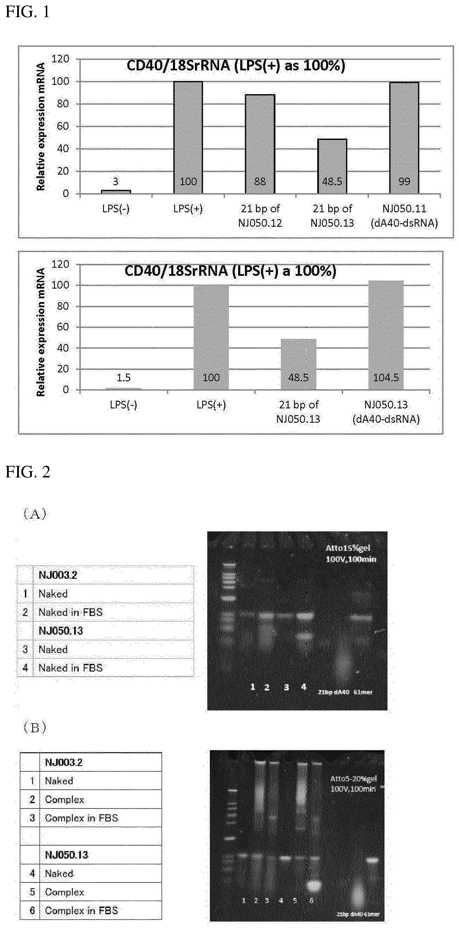

The obtained result is shown in Fig. 1. In Fig. 1, "LPS(-)" denotes the negative control, "LPS(+)" denotes the positive control, "21bp of NJ050.12" denotes the chemically modified siRNA (21 base length) of NJ050.12, "21bp of NJ050.13" denotes the chemically modified siRNA (21 base length) of NJ050.13, "NJ050.11 (dA40-dsRNA)" denotes the SPG/chemically modified siRNA complex obtained by using the chemically modified siRNA of NJ050.11, and "NJ050.13 (dA40-dsRNA)" denotes the SPG/chemically modified siRNA complex obtained by using the chemically modified siRNA of NJ050.13.

From the above results, it has been confirmed that the chemically modified siRNA of NJ050.13 is lower in the amount of CD40 mRNA than the chemically modified siRNA of NJ050.12, indicating that the gene expression inhibitory effect is enhanced when the ribonucleotide residues from the 5' end to the 7th of the antisense strand do not undergo chemical modification. On the other hand, no gene expression inhibitory effect was recognized for the SPG/chemically modified siRNA complex obtained by using the chemically modified siRNA of NJ050.13.

### Reference Test Example 2

As a cause of the fact that no gene expression inhibitory effect was observed for the SPG/chemically modified siRNA complex obtained by using the chemically modified siRNA of NJ050.13, there was a possibility of inadequate uptake into RLC due to vulnerability of the base sequence. Accordingly, in this test, the stability of chemically modified siRNAs and SPG/chemically modified siRNA complexes in serum was evaluated

The chemically modified siRNA (Naked) of NJ050.13 having S-converted polydeoxyadenylic acid (40 mer) linked to the 5' end of the sense strand was added at 1 µM to RPMI1640 medium (Gibco) containing 10% by volume fetal bovine serum (FBS), followed by incubation at 37°C for 16 hours. Then, TE saturated phenol/chloroform was added by the same amount as the sample. The mixture was vigorously stirred by vortex, and then centrifuged at 12,000 × g for 15 minutes. Then, the supernatant was collected, the concentration of nucleic acid in the supernatant was measured with a spectrophotometer, and an amount equivalent to 200 ng of nucleic acid was subjected to electrophoresis using a 15% polyacrylamide gel. As a control, dsRNA of NJ003.2 having 40 S-converted polydeoxyadenylic acids linked to the 5' end of the sense strand by phosphoester bonding was used. The base sequence of the dsRNA of NJ003.2 is as shown in Table 4, and is known to be relatively stable even in serum.

In addition, the test for the SPG/chemically modified siRNA complex obtained by using the chemically modified siRNA of NJ050.13 and the SPG/siRNA complex obtained by using the dsRNA of NJ003.2 was conducted in the same manner as described above, except that the amount of nucleic acid to be used for electrophoresis was set to 400 ng.

The obtained result is shown in Fig. 2. Fig. 2(A) shows the results of the chemically modified siRNA (Naked) of NJ050.13 and the chemically modified siRNA (Naked) of NJ050.13. In Fig. 2(A), "Naked" denotes the case where the incubation was not performed in the presence of FBS, and "Naked in FBS" denotes the case where the incubation was performed in the presence of FBS. Fig. 2(B) shows the results of the SPG/chemically modified siRNA complex obtained by using the chemically modified siRNA of NJ050.13 and the SPG/siRNA complex obtained by using the dsRNA of NJ003.2. In Fig. 2(B), "Naked" denotes the case where the incubation of the chemically modified siRNA of NJ050.13 or the dsRNA of NJ003.2 was not performed in the presence of FBS, "Complex" denotes the case where the incubation of the SPG/chemically modified siRNA complex or SPG/siRNA complex was performed in the presence of FBS, and "Complex in FBS" denotes the case where the incubation of the SPG/chemically modified siRNA complex or SPG/siRNA complex was performed in the presence of FBS.

This result suggests a possibility that the chemically modified siRNA of NJ050.13 having S-converted polydeoxyadenylic acid added to the 5' end of the sense strand may be cleaved near the binding site between polydeoxyadenylic acid and the sense strand.

### Reference Test Example 3

Because the necessity of enhancing the stability near the binding site between the S-converted polydeoxyadenylic acid and the sense strand of siRNA was suggested from the results of Reference Test Example 2, it was decided to chemically modify the bonded site of the sense strand. In addition, because there is a concern that the activity may be lost with the sense strand modification adopted in NJ050.11 from the results of Reference Test Example 1, a chemically modified siRNA of NJ050.14 shown in Table 4 was prepared.

**[Table 4]**

| NJ050.14 |
|---|
| Sense strand 5'→3':C(F)G(F)U(F)G(F)C(F)A(M)G(F)U(F)G(F)A(F)C(F)A(M)A(F)A(M)C(M)A(F)G(F)U(F)A(M)dtdt |
| Antisense strand 5'→3':UACUGUUU(F)G(M)UC(F)A(M)CU(F)G(M)C(F)A(M)CGdtdt |

| |
|---|
| In the Table, "dt", "U(F)", "G(M)" and "A(M)" are as defined in Table 1. |

dRAW cells (mouse macrophages; RAW264 cells overexpressing Dectin-1) were seeded on a 48-well plate at 2 × 10⁴ cells/well, followed by incubation at 37°C under 5% CO2 for 24 hours. Then, the SPG/chemically modified siRNA complex was added at 200 nM or 400 nM, followed by incubation at 37°C under 5% CO2 for 12 hours. Then, interferon γ (IFNr) was added to each well at 0.2 ng/mL, followed by incubation at 37°C under 5% CO2 for 4 hours. Then, the total RNA was extracted from the cells, and cDNA was synthesized from the total RNA. Using the synthesized cDNA as a template, real-time PCR was performed using a CD40 primer to measure the amount of CD40 mRNA. One subjected to the IFNr treatment without addition of the SPG/chemically modified siRNA complex was used as a positive control, and one subjected to neither the IFNr treatment nor addition of the SPG/chemically modified siRNA complex was used as a negative control. The amount of CD40 mRNA was corrected with the amount of 18S rRNA that is a housekeeping gene.

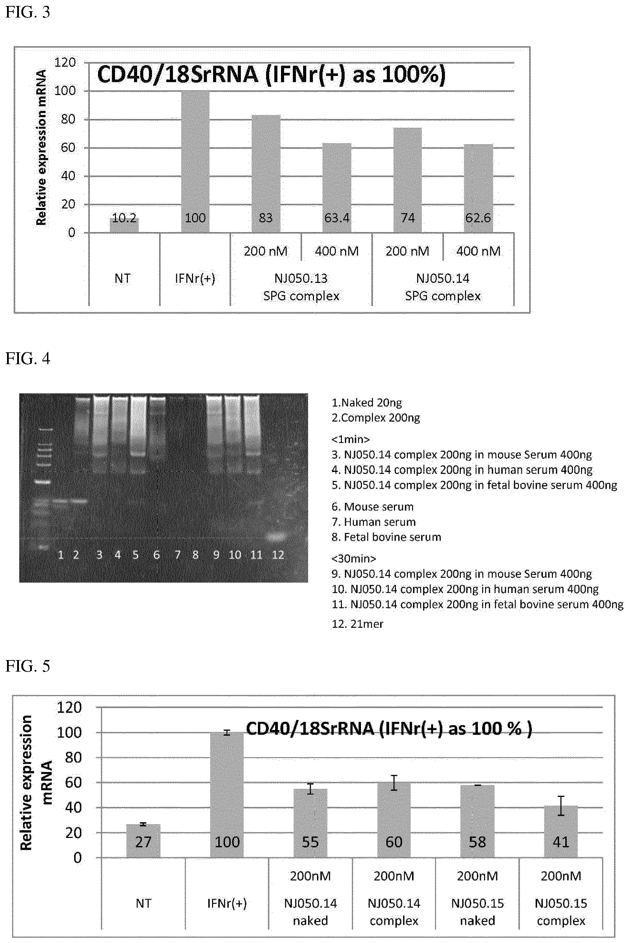

The obtained result is shown in Fig. 3. In Fig. 3, "NT" denotes the negative control, "IFNγ(+)" denotes the positive control, "NJ050.13 SPG complex" denotes the SPG/chemically modified siRNA complex obtained by using the chemically modified siRNA of NJ050.13, "NJ050.14 SPG complex" denotes the SPG/chemically modified siRNA complex obtained by using the chemically modified siRNA of NJ050.14. From the above results, it has been confirmed that the SPG/chemically modified siRNA complex obtained by using the chemically modified siRNA of NJ050.14 has a good gene expression inhibitory effect comparable to the complex obtained by using the chemically modified siRNA of NJ050.13.

### Reference Test Example 4

Added to serum (mouse serum, human serum and FBS) was 200 ng of the SPG/chemically modified siRNA complex obtained by using the chemically modified siRNA of NJ050.14, followed by incubation at 37°C for 1 minute or 30 minutes. Then, TE saturated phenol/chloroform was added by the same amount as the sample. The mixture was vigorously stirred by vortex, and then centrifuged at 12,000 × g for 15 minutes. Then, the supernatant was collected, the concentration of nucleic acid in the supernatant was measured with a spectrophotometer, and an amount equivalent to 200 ng of nucleic acid was subjected to electrophoresis using a 15% polyacrylamide gel.

The obtained result is shown in Fig. 4. In Fig. 4, "Naked" in lane 1 denotes the case where the chemically modified siRNA of NJ050.13 having S-converted polydeoxyadenylic acid added thereto was not incubated in the presence of serum, "Complex" in lane 2 denotes the case where the SPG/chemically modified siRNA complex obtained by using the chemically modified siRNA of NJ050.14 was not incubated in the presence of serum, "NJ050.14 complex" in lanes 3 to 5 and 9 to 11 denotes the SPG/chemically modified siRNA complex obtained by using the chemically modified siRNA of NJ050.14, and "21mer" in lane 12 denotes the sense strand sequence of the chemically modified siRNA moiety of NJ003.2 and was used as a size marker. From this result, it has been confirmed that the SPG/chemically modified siRNA complex obtained by using the chemically modified siRNA of NJ050.14 is relatively stable in the presence of serum.

### Test Example 1

The chemically modified siRNA of NJ050.14 has many sites where the hydroxy group at the 2' position of each of adenylic acid residues and guanylic acid residues is replaced with a methoxy group, and thus there is a concern that the yield may decrease in production. In addition, although the chemically modified siRNA of NJ050.14 is relatively stable in the presence of serum, it is less stable in serum than the chemically modified siRNA of NJ050.13 and thus considered not to endure in vivo use. In view of this, although the chemical modification to the binding site between S-converted polydeoxyadenylic acid and the sense strand of the siRNA is essential, it is necessary to reduce the number of chemical modifications as much as possible even at the expense of stability for development of a chemically modified siRNA having a suitable gene expression inhibitory effect.

In consideration of the above, the chemically modified siRNA of NJ050.15 shown in Table 5 was prepared. The chemically modified siRNA of NJ050.15 satisfies the conditions (i) to (ix) specified in the present invention.

**[Table 5]**

| NJ050.15 (A-3 modification) |
|---|
| Sense strand 5'→3':C(F)GU(F)G(M)C(F)A(M)GU(F)G(M)AC(F)A(M)AAC(F)A(M)GU(F)A(M)dtdt |
| Antisense strand 5'→3':UACUGUUU(F)G(M)UC(F)A(M)CU(F)G(M)C(F)A(M)CGdtdt |

| |
|---|
| In the Table, "dt", "U(F)", "G(M)" and "A(M)" are as defined in Table 1. |

dRAW cells (mouse macrophages; RAW264 cells overexpressing Dectin-1) were seeded on a 48-well plate at 5 × 10⁴ cells/well, and simultaneously, 200 nM of the SPG/chemically modified siRNA complex (obtained by using the chemically modified siRNA of NJ050.14 and NJ050.15) and 0.2 ng/mL of interferon γ (IFNr) were added, followed by incubation at 37°C under 5% CO₂ for 24 hours. Then, the total RNA was extracted from the cells, and cDNA was synthesized from the total RNA. Using the synthesized cDNA as a template, real-time PCR was performed using a CD40 primer to measure the amount of CD40 mRNA. One subjected to the IFNr treatment without addition of the SPG/chemically modified siRNA complex was used as a positive control, and one subjected to neither the IFNr treatment nor addition of the SPG/chemically modified siRNA complex was used as a negative control. The amount of CD40 mRNA was corrected with the amount of 18S rRNA that is a housekeeping gene. For comparison, the same test was performed using the chemically modified siRNAs of NJ050.14 and NJ050.15 having S-converted polydeoxyadenylic acid added thereto.

The obtained result is shown in Fig. 5. In Fig. 5, "NT" denotes the negative control, "IFNγ(+)" denotes the positive control, "NJ050.14 naked" denotes the chemically modified siRNA of NJ050.14 having 40 deoxyadenines linked to the 5' end of the sense strand by phosphoester bonding, "NJ050.14 complex" denotes the SPG/chemically modified siRNA complex obtained by using the chemically modified siRNA of NJ050.14, "NJ050.15 naked" denotes the chemically modified siRNA of NJ050.15 having 40 deoxyadenylic acids linked to the 5' end of the sense strand by phosphoester bonding, and "NJ050.15 complex" denotes the SPG/chemically modified siRNA complex obtained by using the chemically modified siRNA of NJ050.15. From the above results, it has been confirmed that the SPG/chemically modified siRNA complex obtained by using the chemically modified siRNA of NJ050.15 has a comparable or better gene expression inhibitory effect compared to the SPG/chemically modified siRNA complex obtained by using the chemically modified siRNA of NJ050.14.

### Test Example 2

The stability in serum was evaluated for the SPG/chemically modified siRNA complex obtained by using the chemically modified siRNA of NJ050.14 or NJ050.15 and the SPG/chemically modified siRNA complexes obtained by using the chemically modified siRNAs shown in Table 6. Specifically, each of the SPG/chemically modified siRNA complexes was added to serum (human serum and FBS) at 1 µM, followed by incubation at 37°C for 16 hours. Then, TE saturated phenol/chloroform was added by the same amount as the sample. The mixture was vigorously stirred by vortex, and then centrifuged at 12,000 × g for 15 minutes. Then, the supernatant was collected, the concentration of nucleic acid in the supernatant was measured with a spectrophotometer, and an amount equivalent to 20 ng of nucleic acid was subjected to electrophoresis using a 15% polyacrylamide gel. The undegraded chemically modified siRNA was quantified using Image J analysis software to determine the residual ratio (%).

**[Table 6]**

| NJ070.13 |
|---|
| Sense strand 5'→3':C(M)GGUGAAAGCGAAUUCCUAUU |
| Antisense strand 5'→3':UAGGAAUUCGCUUUCACCGUU |

| NJ070.13t |
|---|
| Sense strand 5'→3':C(M)GGUGAAAGCGAAUUCCUAdtdt |
| Antisense strand 5'→3':UAGGAAUUCGCUUUCACCGdtdt |

| NJ070.14 (A-3 modification) |
|---|
| Sense strand 5'→3':C(F)GGU(F)G(M)AAAGCGAAUUCCU(F)A(M)dtdt |
| Antisense strand 5'→3':UAGGAAUUCGCUUUC(F)A(M)CCGdtdt |

| NJ151A3 (A-3 modification) |
|---|
| Sense strand 5'→3':C(F)A(M)U(F)G(M)C(F)A(M)GAGAAAAAC(F)A(M)GU(F)A(M)dtdt |
| Antisense strand 5'→3':UACUGUUUUUCUCU(F)G(M)C(F)A(M)U(F)Gdtdt |

| NJ003.2 |
|---|
| Sense strand 5'→3':GGAGGGCACCGCAGAAUCAUU |
| Antisense strand 5'→3':UGAUUCUGCGGUGCCCUCCUU |

| NJ003.21 (A-3 modification) |
|---|
| Sense strand 5'→3':G(M)GAGGGC(F)A(M)CCGC(F)A(M)GAAUC(F)A(M)dtdt |
| Antisense strand 5'→3':UGAUUCUG(M)CGGU(F)G(M)CCCUCCdtdt |

| |
|---|
| In the Table, "dt", "U(F)", "G(M)" and "A(M)" are as defined in Table 1. |

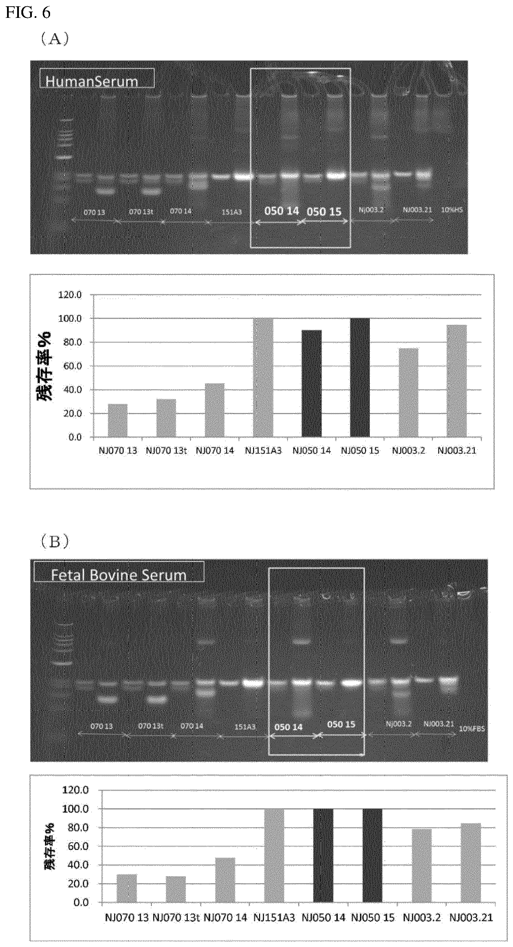

The obtained result is shown in Fig. 6. Fig. 6(A) shows the result obtained by using human serum, and Fig. 6(B) shows the result obtained by using FBS. Surprisingly, the SPG/chemically modified siRNA complex obtained by using the chemically modified siRNA of NJ050.15 had comparable or better stability compared to the complex obtained by using NJ050.14 in spite of its reduced number of chemical modifications.

### Test Example 3

Using the SPG/chemically modified siRNA complex obtained by using the chemically modified siRNA of NJ050.14 or NJ050.15, an x-vivo MLR (mixed lymphocyte culture) assay was performed to evaluate the cell growth suppressing effect. Specifically, the SPG/chemically modified siRNA complex obtained by using the chemically modified siRNA of NJ050.14 or NJ050.15 was administered into BALB/c mice (male, 9 weeks old) via tail vein at 0.2 µg/head or 2 µg/head. After 20 hours, spleens were harvested from the BALB/c mice, and spleen cells (5 × 10⁶ cells/mL) were prepared according to the standard method. The prepared spleen cells were irradiated with 15 Gy radiation for inactivation treatment to prepare stimulator cells.

Separately, spleens were harvested from C57BL/6 mice (male, 9 weeks old), and spleen cells (5 × 10⁶ cells/mL) were prepared according to the standard method to prepare responder cells. In each well of a 96-well plate, 100 µL of each of the stimulator cells and the responder cells were put and subjected to a mixed lymphocyte reaction (MLR) at 37°C under 5% CO₂ for 96 hours. After the reaction, the cells were measured for cell growth using Cell Proliferation ELISA, BrdU kit (Roche Life Science). In place of the SPG/chemically modified siRNA complex, one obtained by using PBS was used as a control.

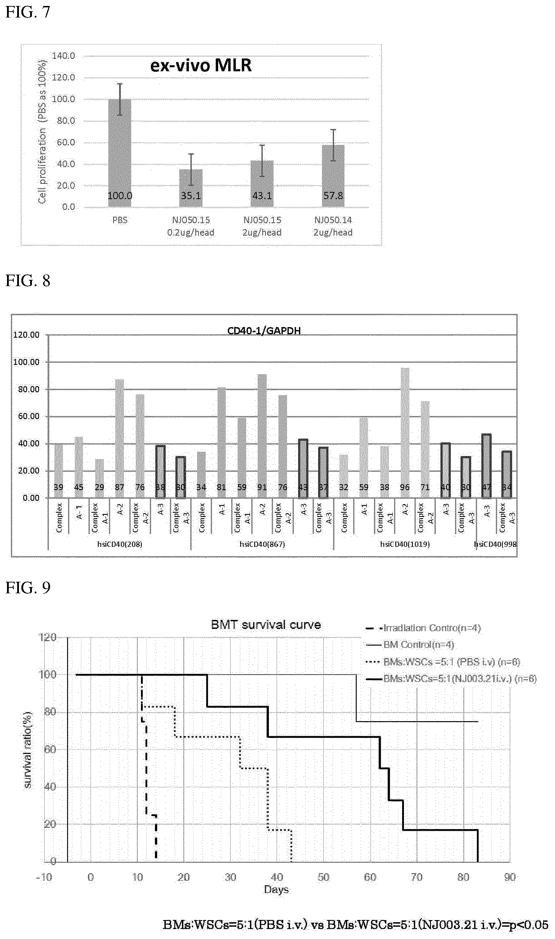

The obtained result is shown in Fig. 7. From this result, it has been confirmed that the SPG/chemically modified siRNA complex obtained by using the chemically modified siRNA of NJ050.15 can improve the stability and induce high knockdown activity with smaller chemical modification.

### Test Example 4

SPG/chemically modified siRNA complexes were prepared using chemically modified siRNAs (siRNAs for CD40, for human sequences) under a specific rule. Specifically, prepared were ones having 40 S-converted deoxyadenylic acids linked to the 5' end of the sense strand of each of the various chemically modified siRNAs shown in Table 7 by phosphoester bonding, to prepare various SPG/chemically modified siRNA complexes in the manner as described above. The base sequences of the sense strand and the antisense strand of hsiCD40 (208) shown in Table 7 correspond to SEQ ID NOs: 3 and 4, respectively, the base sequences of the sense strand and the antisense strand of hsiCD40 (867) correspond to SEQ ID NOs: 5 and 6, respectively, the base sequences of the sense strand and the antisense strand of hsiCD40 (1019) correspond to SEQ ID NOs: 7 and 8, respectively, and the base sequences of the sense strand and the antisense strand of hsiCD40 (998) correspond to SEQ ID NOs: 9 and 10, respectively.

The chemical modification pattern A-1 shown in Table 7 is a modification pattern having relatively high stability and strong activity found as a result of examinations on the stability of complexes of the siRNAs having previously S-converted polydeoxyadenine added thereto with SPG. In the chemical modification pattern A-1, in the sense strand, the hydroxy group at the 2' position of each of the 1, 2, 3, 4, 5, 7, 8, 9, 10, 11, 13, 16, 17 and 18th ribonucleotide residues from the 5' end is replaced with a fluoro group, and the hydroxy group at the 2' position of each of the 6, 12, 14, 15 and 19th ribonucleotide residues from the 5' end is replaced with a methoxy group. In the chemical modification pattern A-1, in the antisense strand, the hydroxy group at the 2' position of each of cytidylic acid residues and uridylic acid residues in a dinucleotide sequence composed of CA, UA and UG is replaced with a fluoro group, and the hydroxy group at the 2' position of each of adenylic acid residues and guanylic acid residues in the dinucleotide sequence is replaced with a methoxy group.

In the chemical modification pattern A-2 shown in Table 7, in the sense strand, the hydroxy group at the 2' position of each of all cytidylic acid residues and uridylic acid residues is replaced with a fluoro group, and the hydroxy group at the 2' position of each of all adenylic acid residues and guanylic acid residues is replaced with a methoxy group. In the chemical modification pattern A-2, in the antisense strand, the hydroxy group at the 2' position of each of cytidylic acid residues and uridylic acid residues in a dinucleotide sequence composed of CA, UA and UG is replaced with a fluoro group, and the hydroxy group at the 2' position of each of adenylic acid residues and guanylic acid residues in the dinucleotide sequence is replaced with a methoxy group. However, the 1st to 7th ribonucleotide residues from the 5' end of the antisense strand are not modified.

The chemical modification pattern A-3 shown in Table 7 satisfies the conditions (i) to (ix) specified in the present invention.

**[Table 7]**

| | Chemical modificatio n pattern | Sequence |
|---|---|---|
| hsiCD40 (208) | A-1 | Sense strand 5'→3':G(F)A(F)C(F)A(F)G(F)A(M)A(F)A(F)C(F)U(F)G(F)G(M)U(F)G(M)A(M)G(F)U(F)G(F)A(M)dtdt |
| | | Antisense strand 5'→3':UCACUCACC(F)A(M)GUUUCU(F)G(M)UCdtdt |
| | A-2 | Sense strand 5'→3':G(M)A(M)C(F)A(M)G(M)A(M)A(M)A(M)C(F)U(F)G(M)G(M)U(F)G(M)A(M)G(M)U(F)G(M)A(M)dtdt |
| | | Antisense strand 5'→3':UCACUCACC(F)A(M)GUUUCU(F)G(M)UCdtdt |
| | A-3 | Sense strand 5'→3':G(M)A(M)C(F)A(M)GAAACU(F)G(M)GU(F)G(M)AGU(F)G(M)Adtdt |
| | | Antisense strand 5'→3':UCACUCACC(F)A(M)GUUUCU(F)G(M)UCdtdt |
| hsiCD40 (867) | A-1 | Sense strand 5'→3':A(F)G(F)U(F)G(F)U(F)G(M)G(F)C(F)C(F)A(F)C(F)G(M)U(F)G(M)G(M)G(F)C(F)A(F)A(M)dtdt |
| | | Antisense strand 5'→3':UUGCCCACGU(F)G(M)GCC(F)A(M)C(F)A(M)CUdtdt |
| | A-2 | Sense strand 5'→3':A(M)G(M)U(F)G(M)U(F)G(M)G(M)C(F)C(F)A(M)C(F)G(M)U(F)G(M)G(M)G(M)C(F)A(M)A(M)dtdt |
| | | Antisense strand 5'→3':UUGCCCACGU(F)G(M)GCC(F)A(M)C(F)A(M)CUdtdt |
| | A-3 | Sense strand 5'→3':A(M)GU(F)G(M)U(F)G(M)GCC(F)A(M)CGU(F)G(M)GGC(F)A(M)Adtdt |
| | | Antisense strand 5'→3':UUGCCCACGU(F)G(M)GCC(F)A(M)C(F)A(M)CUdtdt |
| hsiCD40 (1019) | A-1 | Sense strand 5'→3':C(F)A(F)G(F)A(F)A(F)A(M)C(F)A(F)G(F)U(F)U(F)C(M)A(F)C(M)C(M)U(F)U(F)G(F)A(M)dtdt |
| | | Antisense strand 5'→3':UCAAGGUGAACU(F)G(M)UUUCU(F)Gdtdt |
| | A-2 | Sense strand 5'→3':C(F)A(M)G(M)A(M)A(M)A(M)C(F)A(M)G(M)U(F)U(F)C(F)A(M)C(F)C(F)U(F)U(MG(M)A(M)dtdt |
| | | Antisense strand 5'→3':UCAAGGU(F)G(M)AACU(F)G(M)UUUCU(F)Gdtdt |
| | A-3 | Sense strand 5'→3':C(F)AGA(M)AAC(F)A(M)GUUC(F)A(M)CCUU(F)G(M)Adtdt |
| | | Antisense strand 5'→3':UCAAGGU(F)G(M)AACU(F)G(M)UUUCU(F)Gdtdt |
| hsiCD40 (998) | A-3 | Sense strand 5'→3': C(F)A(M)GGAGACCU(F)G(M)GC(F)A(M)CU(F)G(M)GAdtdt |
| | | Antisense strand 5'→3':UCCAGUGCC(F)A(M)GGUCUCCU(F)Gdtdt |

| | | |
|---|---|---|
| In the Table, "dt", "U(F)", "G(M)" and "A(M)" are as defined in Table 1. | | |

Into 500,000 human peripheral blood mononuclear cells (PBMCs), 400 nM of each of the SPG/chemically modified siRNA complexes was introduced by electroporation. After the introduction, the cells were seeded on a 48-well plate at 2 × 10⁵ cells/well, and simultaneously, TNFα was added at 2.5 ng/mL, followed by incubation at 37°C under 5% CO₂ for 24 hours. Then, the total RNA was extracted from the cells, and cDNA was synthesized from the total RNA. Using the synthesized cDNA as a template, real-time PCR was performed using a CD40 primer to measure the amount of CD40 mRNA. One subjected to the LPS treatment without insertion of siRNA was used as a positive control, and one subjected to neither the LPS treatment nor insertion of siRNA was used as a negative control. The results were analyzed by the ΔΔCT method, and the amount of CD40 mRNA was corrected with the amount of GAPDH that is a housekeeping gene. For comparison, the same test was performed using a SPG/siRNA complex obtained by using a chemically unmodified siRNA and various chemically modified siRNAs each having 40 S-converted deoxyadenylic acids linked to the 5' end of the sense strand by phosphoester bonding, in place of the SPG/chemically modified siRNA complexes.

The obtained result is shown in Fig. 8. In Fig. 8, "Complex" denotes an SPG/siRNA complex obtained by using a chemically unmodified siRNA, "A-1" denotes the chemically modified siRNA subjected to the chemical modification pattern A-1 having 40 S-converted deoxyadenylic acids linked to the 5' end of the sense strand by phosphoester bonding, "Complex A-1" denotes the SPG/chemically modified siRNA complex obtained by using the chemically modified siRNA subjected to the chemical modification pattern A-1, "A-2" denotes the chemically modified siRNA subjected to the chemical modification pattern A-2 having 40 S-converted deoxyadenylic acids linked to the 5' end of the sense strand by phosphoester bonding, "Complex A-2" denotes the SPG/chemically modified siRNA complex obtained by using the chemically modified siRNA subjected to the chemical modification pattern A-2, "A-3" denotes the chemically modified siRNA subjected to the chemical modification pattern A-3 having 40 S-converted deoxyadenylic acids linked to the 5' end of the sense strand by phosphoester bonding, and "Complex A-3" denotes the SPG/chemically modified siRNA complex obtained by using the chemically modified siRNA subjected to the chemical modification pattern A-3. The result indicates that, when the SPG/chemically modified siRNA complex obtained by using the chemically modified siRNA subjected to the chemical modification pattern A-3 was used, the gene expression inhibitory effect was the highest.

From the results of Test Examples 1 to 4, it has been clarified that the SPG/chemically modified siRNA complexes obtained by using the chemically modified siRNAs subjected to the chemical modification satisfying the conditions (i) to (ix) specified in the present invention are high in both resistance to RNase and RNAi activity, and can exert a sufficient gene expression inhibitory effect even in in vivo use.

### Test Example 5

Using the SPG/chemically modified siRNA complex prepared using the chemically modified siRNA subjected to the A-3 chemical modification pattern of hsiCD40 (998) shown in Table 7 (SPG/chemically modified hsiCD40 (998) A-3 complex), a liquid preparation having the composition shown in Table 8 was prepared.

The obtained liquid preparation was sealed in a vial, and a stability test was performed. In the stability test, a long-term storage test was performed by storing the liquid preparation at 5°C in the dark for 6 months and 13 months, and an accelerated test was performed by storing the liquid preparation at 25°C under 60% relative humidity in the dark for 1 month, 3 months and 6 months to measure the properties of the liquid preparation after storage, the content of the SPG/chemically modified hsiCD40 (998) A-3 complex, and the like.

As a result, it has been found that there is no change in the content of the SPG/chemically modified hsiCD40 (998) A-3 complex after storage for both the long-term storage test and the accelerated test, and further there is no change in the properties of the liquid preparation after storage, and thus the liquid preparation is stable.

**[Table 8]**

| Component | Concentration |
|---|---|
| SPG/chemically modified hsiCD40 (998) A-3 complex (in terms of amount of chemically modified siRNA) | 0.4 mg/mL |
| Sodium chloride | 9.0 mg/mL |
| Potassium dihydrogen phosphate | 0.1440 mg/mL |
| Disodium hydrogen phosphate dihydrate | 0.5279 mg/mL |
| Water for injection | Balance |

### Test Example 7

Using the SPG/chemically modified siRNA complex NJ003.21 (A-3 modification) shown in Table 6, evaluation was performed using mouse aGVHD (acute-graft-versus-host-disease) model (recipient: 9 weeks old, female, BALB/C (H2k-d), donor: 9 weeks old, female, C57BL/6N (H2k-b)) as an animal model. In the animal model, the recipient mice are irradiated with X-rays (8.5 Gray) for destruction of the immune system. By transplanting bone marrow cells from the donor mice into the recipient mice whose immune system has been destroyed, the immune system is reconstructed. In this way, bone marrow transplantation is successfully achieved. aGVHD is caused by the transfer of cells other than bone marrow cells of the donor mice.

Specifically, the test was conducted by dividing the mice into 4 groups of BMs: WSCs = 5 : 1 (NJ003.21 i.v.) group (n = 6), Irradiation control group (n = 4), BM control group (n = 4) and BMs : WSCs = 5:1 (PBS i.v.) group (n = 6) under the following conditions.

### <BMs: WSCs = 5:1 (NJ003.21 i.v.) group>

First, the recipient mice were injected via tail vein (i.v.) 3 days and 1 day before cell transplantation (days -3 and -1) such that the SPG/chemically modified siRNA complex was 2 µg/head in terms of the amount of chemically modified siRNA. One day after the date of administration of the second SPG/chemically modified siRNA complex, the recipient mice were irradiated with X-rays (8.5 Gray) for destruction of the immune system. Then, cell transplantation was performed by administering bone marrow cells (5 × 10⁶ cells/mouse) and spleen cells (1 × 10⁶ cells/mouse) collected from the donor mice via tail vein (day 0). The recipient mice were injected via tail vein (i.v.) 1, 4, 6 and 8 days after cell transplantation (days 4, 7, 9 and 11) such that the SPG/chemically modified siRNA complex was 2 µg/head in terms of the amount of chemically modified siRNA. After that, the recipient mice were bred, and the number of survival days was confirmed.

### <BM control group>

The test was conducted under the same conditions as those for the BMs: WSCs = 5:1 (NJ003.21 i.v.) group, except that the SPG/chemically modified siRNA complex was not administered and no cell transplantation was performed.

### <Irradiation control group>

The test was conducted under the same conditions as those for the BMs: WSCs = 5:1 (NJ003.21 i.v.) group, except that the SPG/chemically modified siRNA complex was not administered and the transplanted cells were changed to include only bone marrow cells (5 × 10⁶ cells/mouse).

### <BMs: WSCs = 5:1 (PBS i.v.) group>

The test was conducted under the same conditions as those for the BMs: WSCs = 5:1 (NJ003.21 i.v.) group, except that the same amount of PBS was administered in place of the SPG/chemically modified siRNA complex.

The obtained result is shown in Fig. 9. The result indicates that the survival rate of the BMs: WSCs = 5 : 1 (NJ003.21 i.v.) group was higher than that of the BMs: WSCs = 5 : 1 (PBS i.v.) group, so that the life was prolonged. Therefore, it has been confirmed that NJ003.21 (mouse ortholog of hsiCD40 (998)) is effective for prevention or treatment of aGVHD in mice.

## Claims

1. A pharmaceutical composition comprising a schizophyllan/chemically modified siRNA complex in which a chemically modified siRNA is complexed with schizophyllan:
wherein in the chemically modified siRNA, polydeoxyadenylic acid is added to a 5' end of a sense strand,
a base sequence of the sense strand of the chemically modified siRNA contains at least one dinucleotide sequence selected from the group consisting of CA, UA and UG,
a base sequence of an 8th base and subsequent bases from a 5' end of an antisense strand of the chemically modified siRNA contains at least one dinucleotide sequence selected from the group consisting of CA, UA and UG, and
the chemically modified siRNA satisfies following conditions (i) to (ix):
(i) in the base sequence of the sense strand, when a dinucleotide sequence composed of CA is contained, a hydroxy group at a 2' position of a cytidylic acid residue in the dinucleotide sequence is replaced with a fluoro group, and a hydroxy group at a 2' position of an adenylic acid residue in the dinucleotide sequence is replaced with a methoxy group,
(ii) in the base sequence of the sense strand, when a dinucleotide sequence composed of UA is contained, a hydroxy group at a 2' position of a uridylic acid residue in the dinucleotide sequence is replaced with a fluoro group, and a hydroxy group at a 2' position of an adenylic acid residue in the dinucleotide sequence is replaced with a methoxy group,
(iii) in the base sequence of the sense strand, when a dinucleotide sequence composed of UG is contained, a hydroxy group at a 2' position of a uridylic acid residue in the dinucleotide sequence is replaced with a fluoro group, and a hydroxy group at a 2' position of a guanylic acid residue in the dinucleotide sequence is replaced with a methoxy group,
(iv) in a base sequence of an 8th base and subsequent bases from the 5' end of the antisense strand, when a dinucleotide sequence composed of CA is contained, a hydroxy group at a 2' position of a cytidylic acid residue in the dinucleotide sequence is replaced with a fluoro group, and a hydroxy group at a 2' position of an adenylic acid residue in the dinucleotide sequence is replaced with a methoxy group,
(v) in a base sequence of an 8th base and subsequent bases from the 5' end of the antisense strand, when a dinucleotide sequence composed of UA is contained, a hydroxy group at a 2' position of a uridylic acid residue in the dinucleotide sequence is replaced with a fluoro group, and a hydroxy group at a 2' position of an adenylic acid residue in the dinucleotide sequence is replaced with a methoxy group,
(vi) in a base sequence of an 8th base and subsequent bases from the 5' end of the antisense strand, when a dinucleotide sequence composed of UG is contained, a hydroxy group at a 2' position of a uridylic acid residue in the dinucleotide sequence is replaced with a fluoro group, and a hydroxy group at a 2' position of a guanylic acid residue in the dinucleotide sequence is replaced with a methoxy group,
(vii) 1st to 7th ribonucleotide residues from the 5' end of the antisense strand are not chemically modified,
(viii) when chemical modification is performed according to the above (i) to (vi), in a case where neither a 1st ribonucleotide residue from the 5' end of the sense strand nor a 19th ribonucleotide residue from the 5' end of the antisense strand is chemically modified, if the 1st ribonucleotide residue from the 5' end of the sense strand is a cytidylic acid residue or a uridylic acid residue, a hydroxy group at a 2' position is replaced with a fluoro group, and if the ribonucleotide residue is an adenylic acid residue or a guanylic acid residue, a hydroxy group at a 2' position is replaced with a methoxy group,
(ix) when chemical modification according to the above (i) to (vi) is performed, in a case where both of a 1st ribonucleotide residue from the 5' end of the sense strand and a 19th ribonucleotide residue from the 5' end of the antisense strand are chemically modified, the 19th ribonucleotide residue from the 5' end of the antisense strand is not chemically modified.

2. The pharmaceutical composition according to claim 1, wherein the polydeoxyadenylic acid added to the 5' end of the sense strand of the chemically modified siRNA has a length of 10 to 100 bases.

3. The pharmaceutical composition according to claim 1 or 2, wherein the siRNA is an siRNA for CD40.

4. The pharmaceutical composition according to any one of claims 1 to 3, wherein the chemically modified siRNA contains following sense strand and antisense strand,
Sense strand: 5'-C(F)A(M)GGAGACCU(F)G(M)GC(F)A(M)CU(F)G(M)GAdtdt-3'
Antisense strand: 5'-UCCAGUGCC(F)A(M)GGUCUCCU(F)Gdtdt-3'
[In the sense strand and the antisense strand, "dt" refers to a deoxythymidylic acid residue, "U(F)" refers to a uridylic acid residue in which a hydroxy group at a 2' position is modified with a fluoro group, "G(M)" refers to a guanylic acid residue in which a hydroxy group at a 2' position is replaced with a methoxy group, "C(F)" refers to a cytidylic acid residue in which a hydroxy group at a 2' position is modified with a fluoro group, and "A(M)" refers to an adenylic acid residue in which a hydroxy group at a 2' position is replaced with a methoxy group.].

5. The pharmaceutical composition according to claim 4, wherein the polydeoxyadenylic acid added to the 5' end of the sense strand has a length of 40 bases.

6. The pharmaceutical composition according to any one of claims 1 to 3, wherein the chemically modified siRNA contains following sense strand and antisense strand,
Sense strand: 5'-G(M)A(M)C(F)A(M)GAAACU(F)G(M)GU(F)G(M)AGU(F)G(M)Adtdt-3 '
Antisense strand: 5'-UCACUCACC(F)A(M)GUUUCU(F)G(M)UCdtdt-3'
[In the sense strand and the antisense strand, "dt" refers to a deoxythymidylic acid residue, "U(F)" refers to a uridylic acid residue in which a hydroxy group at a 2' position is modified with a fluoro group, "G(M)" refers to a guanylic acid residue in which a hydroxy group at a 2' position is replaced with a methoxy group, "C(F)" refers to a cytidylic acid residue in which a hydroxy group at a 2' position is modified with a fluoro group, and "A(M)" refers to an adenylic acid residue in which a hydroxy group at a 2' position is replaced with a methoxy group.].

7. The pharmaceutical composition according to claim 6, wherein the polydeoxyadenylic acid added to the 5' end of the sense strand has a length of 40 bases.

8. The pharmaceutical composition according to any one of claims 1 to 3, wherein the chemically modified siRNA contains following sense strand and antisense strand,
Sense strand: 5'-A(M)GU(F)G(M)U(F)G(M)GCC(F)A(M)CGU(F)G(M)GGC(F)A(M)Adtdt-3'
Antisense strand: 5'-UUGCCCACGU(F)G(M)GCC(F)A(M)C(F)A(M)CUdtdt-3'
[In the sense strand and the antisense strand, "dt" refers to a deoxythymidylic acid residue, "U(F)" refers to a uridylic acid residue in which a hydroxy group at a 2' position is modified with a fluoro group, "G(M)" refers to a guanylic acid residue in which a hydroxy group at a 2' position is replaced with a methoxy group, "C(F)" refers to a cytidylic acid residue in which a hydroxy group at a 2' position is modified with a fluoro group, and "A(M)" refers to an adenylic acid residue in which a hydroxy group at a 2' position is replaced with a methoxy group.].

9. The pharmaceutical composition according to claim 8, wherein the polydeoxyadenylic acid added to the 5' end of the sense strand has a length of 40 bases.

10. The pharmaceutical composition according to any one of claims 1 to 3, wherein the chemically modified siRNA contains following sense strand and antisense strand,
Sense strand: 5'-C(F)AGA(M)AAC(F)A(M)GUUC(F)A(M)CCUU(F)G(M)Adtdt-3'
Antisense strand: 5'-UCAAGGU(F)G(M)AACU(F)G(M)UUUCU(F)Gdtdt-3'
[In the sense strand and the antisense strand, "dt" refers to a deoxythymidylic acid residue, "U(F)" refers to a uridylic acid residue in which a hydroxy group at a 2' position is modified with a fluoro group, "G(M)" refers to a guanylic acid residue in which a hydroxy group at a 2' position is replaced with a methoxy group, "C(F)" refers to a cytidylic acid residue in which a hydroxy group at a 2' position is modified with a fluoro group, and "A(M)" refers to an adenylic acid residue in which a hydroxy group at a 2' position is replaced with a methoxy group.].

11. The pharmaceutical composition according to claim 10, wherein the polydeoxyadenylic acid added to the 5' end of the sense strand has a length of 40 bases.

12. The pharmaceutical composition according to any one of claims 1 to 11, being a liquid preparation further comprising sodium chloride, potassium dihydrogen phosphate and disodium hydrogen phosphate.

13. The pharmaceutical composition according to any one of claims 3 to 12, being used for treatment or prevention of resistance or rejection to a transplanted organ, transplanted tissue or transplanted cell.

14. The pharmaceutical composition according to any one of claims 3 to 12, being used for treatment or prevention of graft-versus-host disease.

15. A method for treating or preventing resistance or rejection to a transplanted organ, transplanted tissue or transplanted cell, comprising administering to a patient undergoing an organ, tissue or cell transplantation treatment the pharmaceutical composition according to any one of claims 3 to 12 before and/or after the transplantation treatment.

16. A method for treating or preventing graft-versus-host disease, comprising administering to a patient undergoing an allogeneic hematopoietic stem cell transplantation treatment the pharmaceutical composition according to any one of claims 3 to 12 before and/or after the transplantation treatment.

17. A use of the pharmaceutical composition according to any one of claims 3 to 12 for production of an agent for treating or preventing resistance or rejection to a transplanted organ, transplanted tissue or transplanted cell.

18. A use of the pharmaceutical composition according to any one of claims 3 to 12 for production of an agent for treating or preventing graft-versus-host disease.


SEQUENCE LISTING

<110>  NapaJen Pharma, Incorporated

<120>  Pharmaceutical composition containing a complex of schizophyllan and
chemically modified siRNA

<130>  19029WO

<150>  JP2018-103933
<151>  2017-05-30

<160>  10

<170>  PatentIn version 3.5

<210>  1
<211>  21
<212>  DNA
<213>  Artificial Sequence

<220>
<223>  Sense strand of siRNA for CD40

<400>  1
cgugcaguga caaacaguat t                                                21


<210>  2
<211>  21
<212>  DNA
<213>  Artificial Sequence

<220>
<223>  Antisense strand of siRNA for CD40

<400>  2
uacuguuugu cacugcacgt t                                                21


<210>  3
<211>  21
<212>  DNA
<213>  Artificial Sequence

<220>
<223>  Sense strand of hsiCD40(208)

<400>  3
gacagaaacu ggugagugat t                                                21


<210>  4
<211>  21
<212>  DNA
<213>  Artificial Sequence

<220>
<223>  Antisense strand of hsiCD40(208)

<400>  4
ucacucacca guuucuguct t                                                21

<210>  5
<211>  21
<212>  DNA
<213>  Artificial Sequence

<220>
<223>  Sense strand of hsiCD40(867)

<400>  5
aguguggcca cgugggcaat t                                                21


<210>  6
<211>  21
<212>  DNA
<213>  Artificial Sequence

<220>
<223>  Antisense strand of hsiCD40(867)

<400>  6
uugcccacgu ggccacacut t                                                21


<210>  7
<211>  21
<212>  DNA
<213>  Artificial Sequence

<220>
<223>  Sense strand of hsiCD40(1019)

<400>  7
cagaaacagu ucaccuugat t                                                21


<210>  8
<211>  21
<212>  DNA
<213>  Artificial Sequence

<220>
<223>  Antisense strand of hsiCD40(1019)

<400>  8
ucaaggugaa cuguuucugt t                                                21


<210>  9
<211>  21
<212>  DNA
<213>  Artificial Sequence

<220>
<223>  Sense srrand of hsiCD40(998)

<400>  9
caggagaccu ggcacuggat t                                                21


<210>  10
<211>  21

<212>  DNA
<213>  Artificial Sequence

<220>
<223>  Antisense srrand of hsiCD40(998)

<400>  10
uccagugcca ggucuccugt t                                                21

SEQUENCE LISTING

<110>  NapaJen Pharma, Incorporated

<120>  Pharmaceutical composition containing a complex of schizophyllan and
chemically modified siRNA

<130>  19029WO

<150>  JP2018-103933
<151>  2017-05-30

<160>  10

<170>  PatentIn version 3.5

<210>  1
<211>  21
<212>  DNA
<213>  Artificial Sequence

<220>
<223>  Sense strand of siRNA for CD40

<400>  1
cgugcaguga caaacaguat t                                                21


<210>  2
<211>  21
<212>  DNA
<213>  Artificial Sequence

<220>
<223>  Antisense strand of siRNA for CD40

<400>  2
uacuguuugu cacugcacgt t                                                21


<210>  3
<211>  21
<212>  DNA
<213>  Artificial Sequence

<220>
<223>  Sense strand of hsiCD40(208)

<400>  3
gacagaaacu ggugagugat t                                                21


<210>  4
<211>  21
<212>  DNA
<213>  Artificial Sequence

<220>
<223>  Antisense strand of hsiCD40(208)

<400>  4
ucacucacca guuucuguct t                                                21

<210>  5
<211>  21
<212>  DNA
<213>  Artificial Sequence

<220>
<223>  Sense strand of hsiCD40(867)

<400>  5
   aguguggcca cgugggcaat t                                                21

<210>  6
<211>  21
<212>  DNA
<213>  Artificial Sequence

<220>
<223>  Antisense strand of hsiCD40(867)

<400>  6
uugcccacgu ggccacacut t                                                21

<210>  7
<211>  21
<212>  DNA
<213>  Artificial Sequence

<220>
<223>  Sense strand of hsiCD40(1019)

<400>  7
cagaaacagu ucaccuugat t                                                21

<210>  8
<211>  21
<212>  DNA
<213>  Artificial Sequence

<220>
<223>  Antisense strand of hsiCD40(1019)

<400>  8
ucaaggugaa cuguuucugt t                                                21

<210>  9
<211>  21
<212>  DNA
<213>  Artificial Sequence

<220>
<223>  Sense srrand of hsiCD40(998)

<400>  9
caggagaccu ggcacuggat t                                                21

<210>  10
<211>  21

<212>  DNA
<213>  Artificial Sequence

<220>
<223>  Antisense srrand of hsiCD40(998)

<400>  10
uccagugcca ggucuccugt t                                                21